# EUROPÄISCHE PATENTSCHRIFT

(11) **EP 3 727 274 B1**
(45) Veröffentlichungstag und Bekanntmachung des Hinweises auf die Patenterteilung: **19.07.2023**
(21) Anmeldenummer: 18830800.1
(22) Anmeldetag: 19.12.2018
(51) Int. Cl.: A61J 1/20, A61M 5/145, B65D 83/42

(54) **BEHÄLTER FÜR MEDIZINISCHE FLÜSSIGKEITEN UND VERFAHREN ZUM BEFÜLLEN EINES SOLCHEN BEHÄLTERS**
CONTAINER FOR MEDICAL LIQUIDS AND METHOD FOR FILLING A CONTAINER OF THIS TYPE
RÉCIPIENT POUR LIQUIDES MÉDICAUX ET PROCÉDÉ DE REMPLISSAGE DUDIT RÉCIPIENT

(30) Priorität: 21.12.2017 DE 102017223505
(43) Veröffentlichungstag der Anmeldung: 28.10.2020
(73) Patentinhaber: Vetter Pharma-Fertigung GmbH & Co. KG, 88212 Ravensburg (DE)
(72) Erfinder: HÄRTEL, Ulrich, 88353 Kisslegg (DE)
(74) Vertreter: Kordel, Mattias
(86) Internationale Anmeldenummer: PCT/EP2018/086000
(87) Internationale Veröffentlichungsnummer: WO 2019/122024

(56) Entgegenhaltungen:
- DE-A1-102009 051 945
- US-A- 3 970 219
- US-A- 5 993 425

## Beschreibung

Die Erfindung betrifft einen Behälter für medizinische Flüssigkeiten sowie ein Verfahren zum Befüllen eines solchen Behälters.

Behälter für medizinische Flüssigkeiten sind insbesondere in Form von vorgefüllten Spritzen oder Karpulen bekannt. Diese weisen mindestens einen verlagerbaren Stopfen auf, meist aus einem pharmazeutischen Gummi, der handkraftbetrieben, durch einen motorischen Antrieb oder auch druckbetrieben in einem solchen medizinischen Behälter verlagert werden kann, um eine in dem Behälter angeordnete medizinische Flüssigkeit aus dem Behälter auszutreiben. Insbesondere bei handkraftbetriebenen medizinischen Behältern ergeben sich dabei Unzulänglichkeiten in Ergonomie und Handhabbarkeit. Gerade, wenn ein Patient sich eine Injektion selbst verabreichen soll, ist dies mit einer herkömmlichen Spritze nur in umständlicher Weise und unter unergonomischer Handhaltung möglich. Problematisch ist auch die hochgenaue Bereitstellung und Dosierung kleiner Mengen von insbesondere weniger als 0,5 ml in herkömmlichen vorgefüllten Spritzen oder Karpulen. Insbesondere bei solchen kleinen Mengen ist eine genaue Abmessung des letztlich injizierten Volumens kaum möglich. Mehrfachapplikationen sind mit herkömmlichen Behältern insbesondere aufgrund der Bildung von Luftblasen und der sich daraus ergebenden Gefahr einer Luftembolie problematisch. Pharmazeutische Gummis, die zur Ausbildung gewöhnlicher Stopfen verwendet werden, sind durchlässig für Sauerstoff, sodass sie schlecht zur Lagerung sauerstoffempfindlicher medizinischer Flüssigkeiten, beispielsweise von Adrenalin, geeignet sind. Hinzu kommt, dass solche Stopfen zu einer reibungsarmen Verlagerung insbesondere in einem Glasbehälter ein Gleitmittel, insbesondere Silikonöl, benötigen, wodurch es zu einer an sich unerwünschten Exposition des Patienten mit dem Gleitmittel kommen kann. Weiterhin weisen solche herkömmlichen Behälter Probleme mit Blick auf ihre Dichtigkeit unter Vakuum- und/oder Druckbelastung auf. Da die zum Ausbringen der medizinischen Flüssigkeit in den Stopfen eingeleiteten Kräfte insbesondere bei Handbetrieb naturgemäß schwanken, sind definierte Flussraten der medizinischen Flüssigkeit beim Ausbringen aus dem Behälter kaum zu gewährleisten. Besteht die Notwendigkeit, vor der Injektion eine Luftblase aus dem Behälter zu entfernen, ergibt sich fast zwangsläufig auch ein unerwünschter Austritt von medizinischer Flüssigkeit beim Austreiben der Luftblase. Nicht zuletzt dies trägt mit dazu bei, dass insbesondere kleine Volumina mit herkömmlichen Behältern kaum genau dosierbar sind.

US5993425A zeigt einen bekannten Behälter.

Der Erfindung liegt die Aufgabe zugrunde, einen Behälter für medizinische Flüssigkeiten sowie ein Verfahren zum Befüllen eines solchen Behälters zu schaffen, wobei die genannten Nachteile nicht auftreten.

Die Aufgabe wird gelöst, indem die Gegenstände der unabhängigen Ansprüche geschaffen werden. Vorteilhafte Ausgestaltungen ergeben sich aus den Unteransprüchen.

Die Aufgabe wird insbesondere gelöst, indem ein Behälter für medizinische Flüssigkeiten geschaffen wird, der einen Innenbehälter aufweist, der seinerseits wiederum ein distales Ende und ein proximales Ende aufweist. An dem proximalen Ende ist ein erstes, proximales poröses Trennelement angeordnet. Das erste poröse Trennelement begrenzt ein Aufnahmevolumen zur Aufnahme einer medizinischen Flüssigkeit. Der Behälter weist außerdem einen Außenbehälter auf, in welchem der Innenbehälter mit dem proximalen Ende und zumindest bereichsweise mit dem Aufnahmevolumen angeordnet ist, wobei der Außenbehälter den Innenbehälter gasdicht umgreift, sodass in einem zwischen einer Außenfläche des Innenbehälters und einer Innenfläche des Außenbehälters angeordneten Periphervolumen des Außenbehälters ein Gas unter Überdruck anordenbar ist, wobei insbesondere das Aufnahmevolumen über das erste poröse Trennelement mit dem Periphervolumen in druckübertragender Verbindung steht. Ein Auslasskanalabschnitt des Behälters ist mit dem distalen Ende des Innenbehälters verbunden, wobei der Auslasskanalabschnitt zumindest bereichsweise außerhalb des Außenbehälters angeordnet ist. In dem Auslasskanalabschnitt ist eine Ventileinrichtung anordenbar, vorzugsweise angeordnet, die eingerichtet ist, um in einer Offenstellung eine Fluidverbindung zwischen einer distalen Auslassöffnung des Auslasskanalabschnitts und dem Aufnahmevolumen freizugeben, und in einer Schließstellung die Fluidverbindung zwischen der distalen Auslassöffnung und dem Aufnahmevolumen zu sperren. In dem Aufnahmevolumen kann eine medizinische Flüssigkeit aufgenommen und gelagert werden, die mittels des unter Überdruck in dem Periphervolumen angeordneten Gases, welches über das erste, proximale poröse Trennelement auf die medizinische Flüssigkeit einwirkt, über den Auslasskanalabschnitt und die Ventileinrichtung durch die distale Auslassöffnung ausgetrieben werden kann, wenn die Ventileinrichtung von ihrer Schließstellung in ihre Offenstellung verlagert wird. Hierzu bedarf es keiner Verlagerung eines beweglichen Stopfens in dem Behälter, nämlich weder in dem Innenbehälter noch in dem Außenbehälter. Vielmehr erfolgt das Austreiben der medizinischen Flüssigkeit ausschließlich durch das unter Überdruck in dem Periphervolumen angeordnete Gas. Auf Aktoren oder eine handbetriebene Verlagerung eines verlagerbaren Stopfens kann somit vollständig verzichtet werden, sodass der Behälter ergonomisch, reproduzierbar und in einfacher Weise für eine Injektion verwendet werden kann. Aufgrund des Überdrucks in dem Periphervolumen ergibt sich auch eine definierte Flussrate für die aus dem Behälter austretende medizinische Flüssigkeit. Diese kann mit hoher Genauigkeit über die Ventileinrichtung entnommen werden, sodass auch kleine Volumina hochgenau dosiert werden können. Die definierte Flussrate kann über den Injektionsverlauf zeitlich konstant sein oder in bestimmter Weise variieren. Eine Mehrfachapplikation ist mithilfe der Ventileinrichtung ohne weiteres möglich, indem diese nach der Dosierung eines ersten gewünschten Volumens der medizinischen Flüssigkeit, welches kleiner ist als das Aufnahmevolumen, von der Offenstellung zurück in die Schließstellung verlagert wird, wobei sie anschließend für eine weitere Injektion wieder von der Schließstellung in die Offenstellung verlagert werden kann. Dies kann mehrfach und quasi beliebig oft wiederholt werden, bis die medizinische Flüssigkeit aus dem Aufnahmevolumen vollständig entnommen ist. Abhängig von dem in dem Periphervolumen angeordneten Gas kann der Behälter ohne weiteres auch für sauerstoffempfindliche medizinische Flüssigkeiten, beispielsweise Adrenalin, verwendet werden, insbesondere wenn ein sauerstoffreduziertes oder sogar sauerstofffreies Gas in dem Periphervolumen angeordnet wird.

Unter einer distalen Richtung wird insbesondere eine Richtung verstanden, die in Ausströmrichtung der medizinischen Flüssigkeit aus dem Behälter, insbesondere also bei bestimmungsgemäßer Orientierung des Behälters in Richtung eines Injektionsziels, das heißt insbesondere eines Körpers beispielsweise eines Patienten, weist. Unter einer proximalen Richtung wird eine entgegengesetzte Richtung verstanden, die entgegen der bestimmungsgemäßen Ausströmrichtung der medizinischen Flüssigkeit aus dem Behälter weist.

Vorzugsweise ist an dem distalen Ende des Innenbehälters ein zweites poröses Trennelement angeordnet, wobei das erste poröse Trennelement und das zweite poröse Trennelement das Aufnahmevolumen begrenzen. Die medizinische Flüssigkeit ist dann insbesondere in dem Aufnahmevolumen zwischen dem ersten porösen Trennelement und dem zweiten porösen Trennelement aufnehmbar, vorzugsweise aufgenommen. Sie kann über das zweite, distale poröse Trennelement, den Auslasskanalabschnitt und die Ventileinrichtung durch die distale Auslassöffnung ausgetrieben werden.

Das erste poröse Trennelement schließt den Innenbehälter vorzugsweise an seinem proximalen Ende, insbesondere unmittelbar an seinem proximalen Ende, ab. Vorzugsweise schließt auch das zweite, distale Trennelement den Innenbehälter an seinem distalen Ende, insbesondere unmittelbar an seinem distalen Ende ab, insbesondere wenn der Auslasskanalabschnitt mehrteilig mit dem Innenbehälter ausgebildet ist. Es ist aber auch möglich, dass der Auslasskanalabschnitt einteilig mit dem Innenbehälter angeordnet ist. In diesem Fall setzt sich der Innenbehälter quasi in dem Auslasskanalabschnitt fort, sodass das distale Ende des Innenbehälters in diesem Fall als gedachtes distales Ende aufzufassen ist, welches im Wesentlichen das distale Ende des Aufnahmevolumens darstellt, wobei dieses gedachte distale Ende und damit zugleich eine distale Grenze des Aufnahmevolumens von der distalen Auslassöffnung des Auslasskanalabschnitts - in proximaler Richtung gesehen - beabstandet sind.

Das wenigstens eine poröse Trennelement, nämlich das erste poröse Trennelement und bevorzugt das zweite poröse Trennelement, ist/sind vorzugsweise raumfest relativ zu dem Innenbehälter angeordnet. Insbesondere sind die Trennelemente relativ zu dem Innenbehälter bevorzugt nicht verlagerbar. Vielmehr sind die Trennelemente vorzugsweise an dem Innenbehälter raumfest fixiert. Bei den Trennelementen handelt es sich demnach insbesondere nicht um verlagerbare Stopfen.

Es ist aber alternativ auch möglich, dass wenigstens eines der porösen Trennelemente verlagerbar ist. Insbesondere kann das proximale poröse Trennelement in dem Innenbehälter verlagerbar angeordnet sein und sich bevorzugt während einem Austreiben der medizinischen Flüssigkeit mit deren proximaler Begrenzungsfläche verlagern. Dies kann in besonders günstiger Weise dazu beitragen, die Ausbildung einer Luftblase zu verhindern. Das zweite, distale Trennelement kann alternativ oder zusätzlich lösbar an dem Innenbehälter befestigbar sein, insbesondere zusammen mit dem Auslasskanalabschnitt. Insbesondere kann das zweite Trennelement Teil des Auslasskanalabschnitts oder mit diesem fest verbunden sein. Das zweite Trennelement kann auch in einen Originalitätsverschluss oder in eine Aufsteck-Kanüle integriert sein.

Der Behälter ist vorzugsweise frei von einem verlagerbaren Stopfen; er weist also bevorzugt keinen verlagerbaren Stopfen auf, insbesondere keinen verlagerbaren Stopfen aus einem pharmazeutischen Gummi. Grundsätzlich ist es aber auch möglich, zusätzlich einen verlagerbaren Stopfen, insbesondere zwischen dem ersten Trennelement und dem zweiten Trennelement in dem Innenbehälter, vorzusehen.

Das erste poröse Trennelement und das zweite poröse Trennelement begrenzen das Aufnahmevolumen insbesondere gemeinsam mit einer Wandung, insbesondere einer Innenwandung, des Innenbehälters, wobei die Wandung insbesondere eine innere Mantelfläche bildet, die das Aufnahmevolumen umgreift, und wobei die porösen Trennelemente jeweils stirnseitige Begrenzungen des Aufnahmevolumens bilden.

Bevorzugt ist das Periphervolumen mindestens so groß wie das Aufnahmevolumen. Das Gasvolumen in dem Periphervolumen entspricht also insbesondere mindestens dem Volumen der medizinischen Flüssigkeit in dem Innenbehälter zwischen den porösen Trennelementen, sodass ein fortwährender, ungestörter Ausfluss der medizinischen Flüssigkeit - vorzugsweise mit definierter Flussrate - aus dem Behälter über das gesamte Austreiben des vollständigen Inhalts des Aufnahmevolumens gewährleistet ist.

Dass das Gas unter Überdruck in dem Periphervolumen angeordnet ist, bedeutet insbesondere, dass das Gas unter einem Druck in dem Periphervolumen angeordnet ist, der größer ist als ein Umgebungsdruck des Behälters, insbesondere als ein Normaldruck, vorzugsweise größer als 1013 mbar. Der Überdruck wird bevorzugt so eingestellt, dass ein gewünschtes Austreibverhalten für die medizinische Flüssigkeit aus dem Aufnahmevolumen erreicht wird. Dies betrifft insbesondere eine vorbestimmte Flussrate für die medizinische Flüssigkeit.

Der Auslasskanalabschnitt ist mit dem distalen Ende des Innenbehälters insbesondere strömungstechnisch verbunden, wobei er vorzugsweise von dem Periphervolumen strömungstechnisch getrennt ist. Dies bedeutet insbesondere, dass keine direkte Fluidverbindung zwischen dem Auslasskanalabschnitt einerseits und dem Periphervolumen andererseits besteht. Solch eine Fluidverbindung besteht höchstens vermittelt über bevorzugt das distale Trennelement, das Aufnahmevolumen und das proximale Trennelement, wobei Gas aus dem Periphervolumen in das Aufnahmevolumen eindringen kann, um die medizinische Flüssigkeit auszutreiben, wobei diese wiederum bevorzugt über das distale Trennelement in den Auslasskanalabschnitt und schließlich über die distale Auslassöffnung austreten kann.

Bei einer bevorzugten Ausgestaltung ist vorgesehen, dass der Innenbehälter und/oder der Außenbehälter Glas aufweist/aufweisen, oder aus Glas besteht/bestehen. Hierdurch können insbesondere dauerhaft druckstabile und jedenfalls zu einer äußeren Umgebung des Behälters hin gasundurchlässige Gefäße bereitgestellt werden. Der Innenbehälter und/oder der Außenbehälter können aber auch ein Metall oder eine Metalllegierung, Kunststoff oder Keramik aufweisen oder aus einem Metall oder einer Metalllegierung, aus Kunststoff oder aus Keramik bestehen.

Vorzugsweise ist die Ventileinrichtung als Originalitätsverschluss ausgebildet, sodass ein erstmaliges Öffnen der Ventileinrichtung zu einer irreversiblen Veränderung an derselben führt, die später ohne weiteres erkannt werden kann. Beispielsweise können an der Ventileinrichtung zerbrechbare Stege, Membranen oder dergleichen vorgesehen sein, die bei einer ersten Betätigung zerbrechen oder reißen.

An der distalen Auslassöffnung des Auslasskanalabschnitts kann in bevorzugter Weise eine Kanüle, eine Spritzennadel oder dergleichen fest angeordnet sein. Es ist aber auch möglich, dass die distale Auslassöffnung an einem Anschlusselement des Auslasskanalabschnitts für eine Injektionseinrichtung, beispielsweise für eine feste Kanüle, eine Spritzennadel, ein Infusionsset, beispielsweise einen Infusionsschlauch, oder dergleichen angeordnet ist. Insbesondere kann die distale Auslassöffnung an einem Luer-Lock-Anschluss ausgebildet sein, den der Auslasskanalabschnitt aufweist.

Alternativ oder zusätzlich ist bevorzugt vorgesehen, dass der Innenbehälter langgestreckt ausgebildet ist. Die Erstreckung des Innenbehälters definiert somit insbesondere eine Längsrichtung, die von dem distalen Ende zum proximalen Ende weist.

Alternativ oder zusätzlich ist bevorzugt vorgesehen, dass der Innenbehälter gerade, insbesondere zylindrisch ausgebildet ist. Dies stellt eine besonders einfache und besonders kostengünstig herstellbare Form des Innenbehälters dar. Besonders bevorzugt ist der Innenbehälter kreiszylindrisch ausgebildet.

Alternativ oder zusätzlich ist bevorzugt vorgesehen, dass der Innenbehälter gewunden, insbesondere spiralig ausgebildet ist. Besonders bevorzugt ist der Innenbehälter um eine Achse, die in Richtung seiner längsten Erstreckung weist, herumgewunden. Auf diese Weise kann die Baulänge des Behälters im Vergleich zu einem Behälter mit einem entlang seines Verlaufs gemessen gleich langen, geraden Innenbehälter verkürzt werden.

Gemäß einer Weiterbildung der Erfindung ist vorgesehen, dass der Innenbehälter als Kapillare ausgebildet ist. Dies bedeutet insbesondere, dass die innere, das Aufnahmevolumen begrenzende Mantelfläche in Abhängigkeit von einer Wechselwirkung der medizinischen Flüssigkeit mit dem Material des Innenbehälters, insbesondere der inneren Mantelfläche, sowie der Oberflächenspannung der medizinischen Flüssigkeit so bemessen ist, dass die medizinische Flüssigkeit beim Befüllen des Behälters durch Kapillarkräfte in das Aufnahmevolumen gefördert wird, wobei dieses zugleich vollständig bis zu dem proximalen porösen Trennelement gefüllt wird. Bei einem zylindrischen Innenbehälter mit gegebener Länge ist die innere Mantelfläche insbesondere bestimmt durch den Innendurchmesser oder inneren Radius des Innenbehälters, der dann entsprechend bemessen ist. Die Ausbildung des Innenbehälters als Kapillare bedingt auch einen vergleichsweise kleinen Innendurchmesser, sodass auch ein kleines Aufnahmevolumen sich über eine gut wahrnehmbare Länge des Innenbehälters erstrecken kann, wobei hier in einfacher Weise eine Maßskala angeordnet sein kann, um auch die Dosierung kleinerer Volumina leicht mit bloßem Auge bestimmen zu können. Eine Injektion ist somit leicht zu verfolgen. Insbesondere ist die Länge des Innenbehälter bevorzugt um ein Mehrfaches größer als der Innendurchmesser.

Sind die Kapillarkräfte des Innenbehälters vernachlässigbar, kann gleichwohl insbesondere eine Injektion bei vertikaler Ausrichtung des Behälters möglich.

Der Innenbehälter weist besonders bevorzugt einen Innendurchmesser von weniger als 10 mm, vorzugsweise von weniger als 8 mm, bevorzugt von höchstens 4 mm, bevorzugt von weniger als 4 mm, bevorzugt von höchstens 3 mm, vorzugsweise von weniger als 3 mm, besonders bevorzugt von mindestens 2 mm bis höchstens 3 mm auf.

Gemäß einer Weiterbildung der Erfindung ist vorgesehen, dass der Auslasskanalabschnitt mit dem Innenbehälter einstückig ausgebildet ist. Besonders bevorzugt ist der Auslasskanalabschnitt mit dem Innenbehälter als einteiliges Glasteil ausgebildet, wobei das zweite, distale poröse Trennelement bevorzugt eine Trennung zwischen dem Innenbehälter und dem Auslasskanalabschnitt darstellt, und wobei dieses bevorzugt im Inneren der einteiligen Anordnung aus dem Auslasskanalabschnitt und dem Innenbehälter, von der distalen Auslassöffnung in proximaler Richtung versetzt angeordnet ist.

Alternativ ist bevorzugt vorgesehen, dass der Auslasskanalabschnitt mit dem Innenbehälter mehrteilig ausgebildet ist. Insbesondere kann der Auslasskanalabschnitt als Aufsatz auf den Innenbehälter ausgebildet sein, der insbesondere auf das distale Ende des Innenbehälters fluiddicht aufsteckbar oder in anderer Weise fluiddicht mit dem distalen Ende des Innenbehälters verbindbar ist. Besonders bevorzugt ist der Auslasskanalabschnitt als Kunststoffaufsatz ausgebildet. Dieser kann somit besonders kostengünstig, beispielsweise mittels eines Spritzgussverfahrens, hergestellt sein.

Unabhängig davon, ob der Auslasskanalabschnitt mit dem Innenbehälter einstückig oder mehrteilig ausgebildet ist, weist der Auslasskanalabschnitt vorzugsweise eine Aufnahme für die Ventileinrichtung auf, die insbesondere als den Auslasskanalabschnitt in Querrichtung durchsetzende Bohrung, quer zur Ausströmrichtung der medizinischen Flüssigkeit aus der distalen Auslassöffnung, ausgebildet sein kann.

Gemäß einer Weiterbildung der Erfindung ist vorgesehen, das das erste poröse Trennelement und/oder das zweite poröse Trennelement als Sinterkörper ausgebildet ist/sind. Ein solches poröses Trennelement ist bevorzugt aus einer Vielzahl von Partikeln gebildet, die nur bereichsweise miteinander stoffschlüssig verbunden sind. Ein solcher Sinterkörper wird bevorzugt hergestellt, indem die aneinander bereichsweise anliegenden Partikel auf eine Erweichungstemperatur, vorzugsweise unterhalb einer Schmelztemperatur, erhitzt werden, sodass sie in ihren Randbereichen erweichen oder teilweise schmelzflüssig werden, sodass sie sich insbesondere im Bereich ihrer Berührungspunkte stoffschlüssig miteinander verbinden. Dabei verbinden sich insbesondere nächste Nachbarn der Partikel miteinander bereichsweise. Zwischen den Partikeln verbleiben feine, insbesondere kapillarartig wirkende Kanäle. Ein besonders bevorzugtes Ausführungsbeispiel eines solchen Sinterkörpers ist eine Fritte, wobei das erste poröse Trennelement und/oder das zweite poröse Trennelement insbesondere als Fritte, bevorzugt als Glasfritte oder Keramikfritte, ausgebildet ist/sind.

Es ist aber auch möglich, dass das erste poröse Trennelement und/oder das zweite poröse Trennelement als Filter oder Filtermembran ausgebildet ist/sind, insbesondere mit einem Porendurchmesser von mindestens 0,5 µm bis höchstens 3 µm, vorzugsweise von mindestens 1 µm bis höchstens 2µm, vorzugsweise 1,6 µm. Vorzugsweise weist wenigstens eines der Trennelemente einen Blaspunkt (Bubble Point) von mindestens 0,1 bar bis höchstens 0,7 bar, vorzugsweise von 0,4 bar auf.

Solche Sinterkörper haben Filtereigenschaften, sodass in vorteilhafter Weise in der medizinischen Flüssigkeit vorhandene Partikel entweder bereits beim Befüllen des Behälters mit der medizinischen Flüssigkeit oder aber bei einem nachfolgenden Austreiben der medizinischen Flüssigkeit aus dem Behälter insbesondere durch das distale poröse Trennelement gefiltert werden können. Dies ist besonders günstig in Zusammenhang mit medizinischen Flüssigkeiten zur ophthalmischen Anwendung, da in den Glaskörper des Auges keine oder nur eine geringe Anzahl sehr kleiner Partikel eingebracht werden darf.

Die porösen Trennelemente erfüllen auch folgende wichtige Funktionen: Beim Befüllen des medizinischen Behälters verhindern Kapillarkräfte im Bereich des ersten, proximalen porösen Trennelements ein Heraustreten der medizinischen Flüssigkeit aus dem Aufnahmevolumen in das Periphervolumen, wobei zugleich die einerseits in dem Innenbehälter und andererseits im Bereich des ersten porösen Trennelements wirksamen Kapillarkräfte bewirken, dass eine vollständige, luftblasenfreie Befüllung des Aufnahmevolumens erfolgt. Weiterhin hat das erste poröse Trennelement die Aufgabe, den Überdruck des Gases in dem Periphervolumen an die Flüssigkeit weiterzuleiten, sodass diese beim Öffnen der Ventileinrichtung über die distale Auslassöffnung ausgetrieben werden kann. Das distale poröse Trennelement verhindert sehr effizient eine Injektion von Luftblasen, da ein Austreten der medizinischen Flüssigkeit über das distale poröse Trennelement stoppt, sobald dieses nicht mehr in Kontakt mit in dem Aufnahmevolumen angeordneter Flüssigkeit, sondern mit einer dort vorhandenen Luftblase oder Gas kommt. Eine unerwünschte Gasinjektion kann daher wirksam vermieden werden.

Vorzugsweise weisen das erste Trennelement und das zweite Trennelement voneinander verschiedene Porengrößen oder Porendurchmesser auf, um zu gewährleisten, dass einerseits Gas durch das erste, proximale Trennelement hindurchtreten kann, um die Injektion zu bewirken, wobei andererseits eine Injektion von Gas durch das zweite, distale Trennelement verhindert wird. Insbesondere weisen das erste Trennelement und das zweite Trennelement verschiedene Blaspunkte (Bubble Point) und somit verschiedene Durchtrittseigenschaften für Gase auf.

Da aber der Druck in dem Periphervolumen im Verlauf der Injektion nachlässt, ist es auch möglich, dass die Trennelemente gleiche Porendurchmesser und/oder Blaspunkte aufweisen, wobei gleichwohl das erste Trennelement bei dem höheren Gasdruck zu Beginn der Injektion einen Gasdurchtritt ermöglicht, wobei das zweite Trennelement zum Ende der Injektion bei dem dann geringeren Gasdruck einen Gasdurchtritt verhindert.

Sind die porösen Trennelemente als Glasfritten ausgebildet, sind sie bevorzugt in den Innenbehälter eingeschmolzen. Dies ist in besonders stabiler und günstiger Weise möglich, wenn auch der Innenbehälter Glas aufweist oder aus Glas besteht.

Eine Ausbildung des Innenbehälters aus Glas hat den zusätzlichen Vorteil, dass dieser eine hohe chemische Inertheit gegenüber der medizinischen Flüssigkeit aufweist. Da kein verlagerbarer Stopfen in dem Innenbehälter vorgesehen ist, bedarf es keiner Verwendung eines Gleitmittels, insbesondere keines Silikonöls. Die medizinische Flüssigkeit kann so ohne jedes Kontaminationsrisiko unter hoher Reinheit in dem Innenbehälter aufbewahrt werden.

Gemäß einer Weiterbildung der Erfindung ist vorgesehen, dass das distale poröse Trennelement außerhalb des Außenbehälters angeordnet ist. Eine Verbindungsstelle, an welcher der Außenbehälter gasdicht mit dem Innenbehälter verbunden, vorzugsweise verschmolzen ist, ist demnach in Bezug auf das distale poröse Trennelement in proximaler Richtung versetzt angeordnet. Es ist aber auch möglich, dass das distale poröse Trennelement innerhalb des Außenbehälters oder gerade im Bereich der Verbindungsstelle zwischen dem Außenbehälter und dem Innenbehälter angeordnet ist.

Gemäß einer Weiterbildung der Erfindung ist vorgesehen, dass der Außenbehälter zylindrisch ausgebildet ist. Dies stellt eine einfache und insbesondere einfach herstellbare Gestaltung des Außenbehälters dar.

Alternativ oder zusätzlich ist bevorzugt vorgesehen, dass der Außenbehälter tonnenförmig oder kolbenförmig ausgebildet ist. Dies bedeutet insbesondere, dass der Außenbehälter eine in zwei aufeinander senkrecht stehenden Richtungen gekrümmte Wandung aufweisen kann. Eine solche Ausgestaltung trägt dazu bei, scharfwinklige Übergänge zu vermeiden, sodass der Außenbehälter besonders druckstabil ausgebildet sein kann.

Alternativ oder zusätzlich ist der Außenbehälter bevorzugt konvex ausgebildet, wobei sich die Angabe der konvexen Ausbildung auf die Blickrichtung eines Betrachters des Außenbehälters von außen bezieht. Die Außenwandung des Außenbehälters weist somit insbesondere global eine nach außen gerichtete Krümmung oder Ausbeulung auf. Auch dies trägt zu einem besonders druckstabilen Außenbehälter bei.

Alternativ oder zusätzlich ist bevorzugt vorgesehen, dass der Außenbehälter ovoid oder oval ausgebildet ist. Dies bedingt eine besonders günstige, sehr druckstabile Form des Außenbehälters, der insbesondere quasi als ei- oder glühbirnenkolbenförmiger Körper ausgebildet sein kann.

Gemäß einer Weiterbildung der Erfindung ist vorgesehen, dass der Auslasskanalabschnitt in einem endlichen, von 0° verschiedenen Winkel zu einer Haupterstreckungsrichtung des Innenbehälters angeordnet ist. Insbesondere erstreckt sich eine Längsrichtung des Auslasskanalabschnitts, das heißt insbesondere eine Auslassrichtung oder Längsrichtung eines Auslassabschnitts, welcher die distale Auslassöffnung aufweist, des Auslasskanalabschnitts in einem endlichen, von 0° verschiedenen Winkel zu der Haupterstreckungsrichtung des Innenbehälters. Die Haupterstreckungsrichtung des Innenbehälters ist insbesondere eine Zylinderachse oder Längsachse des Innenbehälters, wenn dieser zylindrisch ausgebildet ist, oder eine Richtung der längsten Ausdehnung des Innenbehälters, um welche der Innenbehälter beispielsweise zumindest bereichsweise gewunden, insbesondere spiralig gewunden sein kann.

Indem der Auslasskanalabschnitt einen von 0° verschiedenen Winkel mit der Haupterstreckungsrichtung des Innenbehälters einschließt, kann der Behälter insgesamt quasi eine Pistolenform aufweisen, sodass einerseits der Behälter mit einer Hand eines Benutzers einfach und ergonomisch gehalten werden kann, wobei andererseits die Ventileinrichtung, insbesondere mit einem Finger der den Behälter haltenden Hand, einfach und ergonomisch, mit natürlicher Handhaltung, bedient werden kann. Auf diese Weise ist es einem Patienten leicht und ohne größere Anstrengung möglich, sich mit dem Behälter selbst eine Injektion zu verabreichen.

Gemäß einer Weiterbildung der Erfindung ist vorgesehen, dass der Behälter einen Fingerabzug aufweist, der mit der Ventileinrichtung derart wirkverbunden ist, dass die Ventileinrichtung mittels des Fingerabzugs betätigbar ist. Auf diese Weise ist die Ventileinrichtung besonders ergonomisch durch einen Benutzer mit seinem Finger betätigbar. Besonders bevorzugt ist dabei, dass der Auslasskanalabschnitt in einem endlichen, von 0° verschiedenen Winkel zu der Haupterstreckungsrichtung des Innenbehälters angeordnet ist, wobei zugleich der Behälter den Fingerabzug aufweist, mit dem die Ventileinrichtung betätigbar ist. Der Fingerabzug wirkt dabei wie ein Pistolenabzug, der leicht und ergonomisch bedient werden kann. Dies hat große Vorteile insbesondere im Hinblick auf eine einfache, unkomplizierte Selbstinjektion eines Patienten. Der Fingerabzug kann insbesondere ein einfaches, gebogenes Abkrümmelement wie eine Pistole aufweisen. Es ist aber auch möglich, dass der Fingerabzug einen Fingerring, insbesondere einen geschlossenen Fingerring, aufweist, durch welchen der Benutzer des Behälters seinen Finger hindurchstrecken kann. Der Fingerring ermöglicht in besonders einfacher Weise neben einem Öffnen der Ventileinrichtung auch ein Schließen durch Zurückverlagern des Fingerabzugs, wobei kein Umgreifen erforderlich ist.

Gemäß einer Weiterbildung der Erfindung ist vorgesehen, dass die Ventileinrichtung ein Rückschlagventil, ein handbetätigtes Ventil, ein Schaltventil, und/oder ein Kombinationsventil aus einem Rückschlagventil und einem handbetätigten Ventil aufweist. Ein Rückschlagventil erweist sich insbesondere als vorteilhaft beim Befüllen des Behälters, wobei die medizinische Flüssigkeit unter einem Druck, welcher den Überdruck des Gases leicht übersteigt, in das Aufnahmevolumen eingebracht werden kann, wobei in diesem Funktionszustand das Rückschlagventil öffnet. Im normalen Lagerzustand ist dann das Gas in dem Periphervolumen und die medizinische Flüssigkeit in dem Aufnahmevolumen unter dem Überdruck gehalten, der das Rückschlagventil gegen den in einer äußeren Umgebung des Behälters wirkenden Normaldruck in seinen Dichtsitz drängt.

Ein handbetätigtes Ventil kann ohne weiteres durch einen Benutzer geöffnet und geschlossen werden, sodass eine Abgabe der medizinischen Flüssigkeit über die distale Auslassöffnung leicht steuerbar ist. Hierzu kann aber auch ein Schaltventil Anwendung finden, insbesondere wenn die Ventileinrichtung eine zur Ansteuerung eines solchen Schaltventils ausgebildete elektronische Steuereinrichtung aufweist.

Besonders bevorzugt wird ein Kombinationsventil aus einem Rückschlagventil und einem handbetätigten Ventil, wobei das Kombinationsventil sowohl die Funktion einer einfachen Befüllung und eines sicheren Haltens der medizinischen Flüssigkeit in dem Behälter durch die Komponente des Rückschlagventils erfüllt, wobei sie zugleich die Funktion des einfachen Öffnens und Schließens der Ventileinrichtung mit der handbetätigten Ventilkomponente erfüllt. Die handbetätigte Ventilkomponente und das Rückschlagventil sind bevorzugt integral miteinander ausgebildet. Besonders bevorzugt weist das handbetätigte Ventil einen Sitz für das Rückschlagventil auf, wobei dieser Sitz zugleich einen Strömungskanal für die Fluidverbindung zwischen dem Aufnahmevolumen und der distalen Auslassöffnung bereitstellt. Insbesondere kann das handbetätigte Ventil eine Aussparung aufweisen, die in der Schließstellung einen Sitz für ein Ventilelement des Rückschlagventils aufweist, wobei diese Aussparung in der Offenstellung so verschwenkt ist, dass sie als Kanal für die Fluidverbindung zwischen dem Aufnahmevolumen und der distalen Auslassöffnung wirkt.

Insbesondere ist es möglich, dass das handbetätigte Ventil einen um eine schräg, vorzugsweise senkrecht zur Auslassrichtung der medizinischen Flüssigkeit aus dem Behälter angeordneten, schwenkbaren Zylinder aufweist, der eine Aussparung aufweist, die in der Schließstellung in Richtung des Aufnahmevolumens verschwenkt und ausgebildet ist, um einen Dichtsitz für das Ventilelement des Rückschlagventils zu bilden. In der Offenstellung ist diese Aussparung - vorzugsweise um 90° - so verschwenkt, dass ein Fluidpfad zwischen dem Aufnahmevolumen und der distalen Auslassöffnung über diese Aussparung freigegeben ist. Das Ventilelement des Rückschlagventils wird dabei zugleich durch die im Übrigen nicht ausgesparte äußere Umfangsfläche des Zylinders des handbetätigten Ventils entgegen seiner Dichtrichtung in Richtung des Aufnahmevolumens gedrängt, sodass auch hier die Fluidverbindung zwischen dem Aufnahmevolumen und der distalen Auslassöffnung freigegeben ist. Wird der Zylinder zurück in die Schließstellung geschwenkt, kann das Ventilelement des Rückschlagventils wiederum dicht in der Aussparung aufgenommen werden, sodass die Fluidverbindung zwischen dem Aufnahmevolumen und der distalen Auslassöffnung wiederum gesperrt ist.

Die Ventileinrichtung weist vorzugsweise einen Kunststoff auf oder ist aus einem Kunststoff gebildet. Dabei kann es sich um Polyetheretherketon (PEEK) handeln, wobei dieses Material zum einen inert und zum anderen sehr gleitfähig ist, wobei es auch eine gute Dichtwirkung bereitstellt. Es bedarf dann gegebenenfalls keiner zusätzlichen Gleit- und/oder Dichtmittel für die Ventileinrichtung. Es ist auch möglich, dass beispielsweise der Zylinder des handbetätigten Ventils mit O-Ringen gedichtet, und vorzugsweise gehalten und geführt ist. Zusätzlich oder alternativ ist es möglich, dass wenigstens ein wachsartiges Dichtmittel im Bereich der Ventileinrichtung zum Einsatz kommt, um den Behälter gegenüber einem Äußeren insbesondere in der Geschlossenstellung des Ventils zu dichten, und/oder um die Fluidverbindung zwischen dem Aufnahmevolumen und der distalen Auslassöffnung derart gegenüber dem Äußeren des Behälters zu dichten, dass die medizinische Flüssigkeit ausschließlich über die distale Auslassöffnung und nicht etwa durch irgendwelche Spalte im Bereich der Ventileinrichtung seitlich austreten kann.

Es ist möglich, dass das Ventilelement des Rückschlagventils in seine Schließstellung vorgespannt ist, beispielsweise mit einem Federelement oder einem anderen geeigneten Vorspannelement. Es ist aber auch möglich, dass die Ventileinrichtung kein solches Vorspannelement aufweist, wobei die Druckdifferenz zwischen dem Druck in dem Aufnahmevolumen und dem Periphervolumen einerseits und dem Druck in der äußeren Umgebung des Behälters andererseits genügt, um das Ventilelement des Rückschlagventils dicht in seinen Sitz zu drängen.

Gemäß einer bevorzugten Ausgestaltung ist auch die weitere oder anders ausgestaltete Ventileinrichtung in ihre Schließrichtung vorgespannt. Beispielsweise ist es möglich, dass das handbetätigte Ventil - unabhängig ob es für sich oder in Kombination mit dem Rückschlagventil als Kombinationsventil ausgebildet ist - in seine Schließstellung vorgespannt ist, beispielsweise mittels eines Vorspannelements, insbesondere einer Feder, das an einem Betätigungshebel oder dem zuvor beschriebenen Fingerabzug angreift. Ein Öffnen der Ventileinrichtung erfolgt dann gegen die Vorspannkraft, wobei das Schließen der Ventileinrichtung vorzugsweise automatisch erfolgt, sobald ein Benutzer die Ventileinrichtung loslässt oder freigibt.

Gemäß einer Weiterbildung der Erfindung ist vorgesehen, dass der Innenbehälter mit einer medizinischen Flüssigkeit gefüllt ist, wobei der Außenbehälter mit einem Gas unter Überdruck gefüllt ist. Dabei verwirklichen sich die insoweit bereits beschriebenen Vorteile. Insbesondere ist es möglich, dass der Innenbehälter mit einer sauerstoffempfindlichen medizinischen Flüssigkeit gefüllt ist, die dann gefahrlos auch langfristig in dem Behälter gelagert werden kann, wenn als Gas in dem Außenbehälter ein sauerstoffarmes oder sauerstofffreies Gas verwendet wird. Als Gas in dem Außenbehälter kann grundsätzlich jedes Gas verwendet werden, welches nicht mit der medizinischen Flüssigkeit reagiert, beispielsweise Luft, Stickstoff, ein Inertgas, insbesondere ein inertes Edelgas.

Gemäß einer Weiterbildung der Erfindung ist vorgesehen, dass das erste, proximale poröse Trennelement mit einem Abdeckelement zumindest bereichsweise gegenüber dem Periphervolumen des Außenbehälters abgedeckt ist, wobei das Abdeckelement das proximale poröse Trennelement bevorzugt bis auf eine zentrale Aussparung abdeckt. Das Abdeckelement ermöglicht in vorteilhafter Weise eine Verringerung der Kontaktfläche zwischen dem unter Überdruck stehenden Gas und der medizinischen Flüssigkeit, wodurch insbesondere Verdampfungsverluste aus dem Aufnahmevolumen durch das erste, proximale poröse Trennelement hindurch in das Periphervolumen minimiert werden können. Zugleich bietet das Abdeckelement Schutz vor mechanischer Belastung des proximalen porösen Trennelements, sowie insbesondere einen Spritzschutz, wenn der Behälter einer Erschütterung ausgesetzt wird, beispielsweise wenn er versehentlich fallengelassen wird.

Das Abdeckelement ist bevorzugt als Abdeckkappe mit einer insbesondere zentralen Bohrung ausgebildet. Alternativ ist es auch möglich, dass das Abdeckelement als Membran, vorzugsweise mit einer zentralen Aussparung, ausgebildet ist.

Alternativ oder zusätzlich zu einem Abdeckelement ist es auch möglich, dass das erste poröse Trennelement einen Außendurchmesser aufweist, der kleiner ist als der freie Innendurchmesser des Aufnahmevolumens außerhalb des proximalen Endes des Innenbehälters. Insbesondere kann die innere Mantelfläche des Innenbehälters im Bereich des proximalen Endes nach innen aufgedickt sein, mithin radial nach innen vorspringen, wobei das erste poröse Trennelement im Bereich der radialen Aufdickung angeordnet ist. Auch auf diese Weise kann die Kontaktfläche zwischen dem Gas und der medizinischen Flüssigkeit verringert, insbesondere minimiert werden.

Gemäß einer Weiterbildung der Erfindung ist vorgesehen, dass an der distalen Verbindungsstelle, an welcher der Außenbehälter gasdicht mit dem Innenbehälter verbunden, insbesondere mit diesem verschmolzen ist, eine Sollbruchstelle angeordnet ist. Die Sollbruchstelle ist bevorzugt so ausgestaltet, dass sie bei einer Belastung des Behälters durch Schlag, insbesondere durch Aufschlag beim Fallen, ein kontrolliertes Entweichen des Gases ohne Knall und ohne Austritt der medizinischen Flüssigkeit ermöglicht. Die Sollbruchstelle kann bevorzugt als Loch oder Bohrung, insbesondere quer zu einer Längsachse des Behälters, ausgebildet sein. Falls Gas quer zu der Längsachse entweicht, führt dies höchstens zu einer Rotation des Behälters, nicht jedoch zu einer translatorischen Beschleunigung desselben, wodurch insbesondere vermieden wird, dass der Behälter ähnlich einer Rakete beschleunigt wird.

Gemäß einer Weiterbildung der Erfindung ist vorgesehen, dass der Behälter als Spritze oder Karpule ausgebildet ist. Dabei verwirklichen sich in besonderer Weise die bereits beschriebenen Vorteile des Behälters.

Bevorzugt hat das Aufnahmevolumen ein Hohlmaß von weniger als 2 ml, vorzugsweise von weniger als 1,5 ml, vorzugsweise von weniger als 1 ml, vorzugsweise von weniger als 0,6 ml, vorzugsweise von weniger als 0,5 ml, wobei es in günstiger Weise eingerichtet ist, um auch kleine Volumina zur genauen Injektion bereitzustellen. Bevorzugt hat das Aufnahmevolumen ein Hohlmaß von 1 ml. Besonders bevorzugt ist der Behälter eingerichtet für eine ophthalmische Injektion, insbesondere in den Glaskörper eines Auges.

Der Behälter ist weiterhin bevorzugt eingerichtet zur Durchführung einer Mehrfach-Injektion, wobei die Ventileinrichtung bedarfsgerecht geöffnet und geschlossen werden kann, und wobei die Mehrfachinjektion beispielsweise in verschiedene Stellen, insbesondere bei subkutaner oder intramuskulärer Applikation, und/oder über einen längeren Zeitraum, beispielsweise über einen Monat hinweg, erfolgen kann.

Besonders bevorzugt ist der Behälter eingerichtet zur Durchführung einer subkutanen oder intramuskulären Injektion.

Die Aufgabe wird auch gelöst, indem ein Verfahren zum Befüllen eines Behälters für eine medizinische Flüssigkeit nach einem der zuvor beschriebenen Ausführungsbeispiele geschaffen wird, wobei das Verfahren folgende Schritte aufweist: Das Periphervolumen des Behälters wird mit einem Gas unter einem ersten vorbestimmten Druck befüllt. Anschließend wird das Aufnahmevolumen des Innenbehälters mit einer medizinischen Flüssigkeit unter einem zweiten vorbestimmten Druck befüllt, wobei der zweite vorbestimmte Druck größer ist als der erste vorbestimmte Druck, und schließlich wird die Ventileinrichtung geschlossen. Auf diese Weise kann insbesondere das Aufnahmevolumen des Innenbehälters sicher, reproduzierbar und vollständig gefüllt werden.

Dabei wirkt es sich besonders vorteilhaft aus, wenn der Innenbehälter als Kapillare ausgebildet ist, wobei die Kapillarkräfte die Säule der medizinischen Flüssigkeit in dem Aufnahmevolumen beim Befüllen aufbauen, und zugleich später beim Entleeren zusammenhalten, sodass eine Gasinjektion wirksam vermieden wird.

Das Periphervolumen wird vorzugsweise über den Auslasskanalabschnitt und die Ventileinrichtung sowie den Innenbehälter und das erste, proximale poröse Trennelement befüllt. Insbesondere ist das Periphervolumen des Außenbehälters überhaupt nur auf diesem Weg für die Befüllung zugänglich, während der Außenbehälter im Übrigen vollständig gasdicht ausgebildet ist. Auch die Befüllung des Aufnahmevolumens erfolgt über den Auslasskanalabschnitt und die Ventileinrichtung. Auch zu dem Aufnahmevolumen besteht bevorzugt kein anderer Zugangsweg.

Wird als Gas ein anderes Gas als Luft verwendet, wird bevorzugt das Periphervolumen des Behälters - insbesondere über den Auslasskanalabschnitt und die Ventileinrichtung - mehrfach evakuiert und mit reinem, zu verwendenden Gas gefüllt, sodass das Periphervolumen hinreichend gespült wird. Auf diese Weise kann insbesondere eine sauerstoffarme oder sauerstofffreie Atmosphäre in dem Periphervolumen bereitgestellt werden.

Der erste vorbestimmte Druck entspricht bevorzugt einem vorbestimmten Enddruck für das spätere Austreiben der medizinischen Flüssigkeit aus dem Behälter. Im Ergebnis stellt sich nach dem Schließen der Ventileinrichtung in dem Behälter der zweite vorbestimmte Druck als Initialdruck ein.

Zur Befüllung wird der Behälter zumindest mit dem Auslasskanalabschnitt und insbesondere mit der distalen Auslassöffnung bevorzugt in die medizinische Flüssigkeit eingetaucht oder mit einem Druckanschluss zur Versorgung mit der medizinischen Flüssigkeit verbunden. Die Befüllung erfolgt dann automatisch aufgrund des zweiten vorbestimmten Drucks, der größer ist als der erste vorbestimmte Druck, und vorzugsweise aufgrund der in dem Innenbehälter wirksamen Kapillarkräfte. Diese sorgen auch für eine vollständige und blasenfreie Füllung des Aufnahmevolumens, wobei die Befüllung automatisch stoppt, sobald die Kapillarkräfte das Heraustreten der medizinischen Flüssigkeit über das proximale, poröse Trennelement in das Periphervolumen hinaus verhindern.

Wird der äußere, zweite vorbestimmte Druck weggenommen, schließt das Rückschlagventil der Ventileinrichtung bevorzugt automatisch. Eine weitere Ventilkomponente der Ventileinrichtung, bevorzugt ein handbetätigtes Ventil, kann nun entweder zusätzlich erst montiert werden, oder sie wird - sofern sie bereits vor der Befüllung montiert wurde - nach der Befüllung verschlossen.

Beim Befüllen oder vor dem Befüllen des Periphervolumens mit dem Gas wird vorzugsweise in dem Gas ein Partialdruck zumindest eines Hauptbestandteils der medizinischen Flüssigkeit, insbesondere eines Lösungsmittels derselben, so eingestellt, dass der Partialdruck gerade dem Dampfdruck zumindest dieses Hauptbestandteils der medizinischen Flüssigkeit oder der medizinischen Flüssigkeit in dem Innenbehälter bei dem zweiten vorbestimmten Druck und damit dem dauerhaften Lagerdruck des Behälters 1 entspricht. Auf diese Weise wird in dem Periphervolumen insbesondere der Sättigungsdampfdruck der medizinischen Flüssigkeit unter Lagerbedingungen eingestellt. Dies trägt in vorteilhafter Weise dazu bei, ein Verdampfen zumindest von Bestandteilen der medizinischen Flüssigkeit über das proximale poröse Trennelement in das Periphervolumen zu verhindern, wodurch insbesondere verhindert wird, dass das proximale poröse Trennelement austrocknet.

Die Erfindung wird im Folgenden anhand der Zeichnung näher erläutert. Dabei zeigen:
- Figur 1: eine schematische Darstellung eines ersten Ausführungsbeispiels eines Behälters für medizinische Flüssigkeiten;
- Figur 2: eine schematische Darstellung eines zweiten Ausführungsbeispiels eines solchen Behälters;
- Figur 3: eine Detaildarstellung eines Auslasskanalabschnitts eines dritten Ausführungsbeispiels eines solchen Behälters, und
- Figur 4: eine schematische Detaildarstellung eines vierten Ausführungsbeispiels eines Behälters für medizinische Flüssigkeiten.

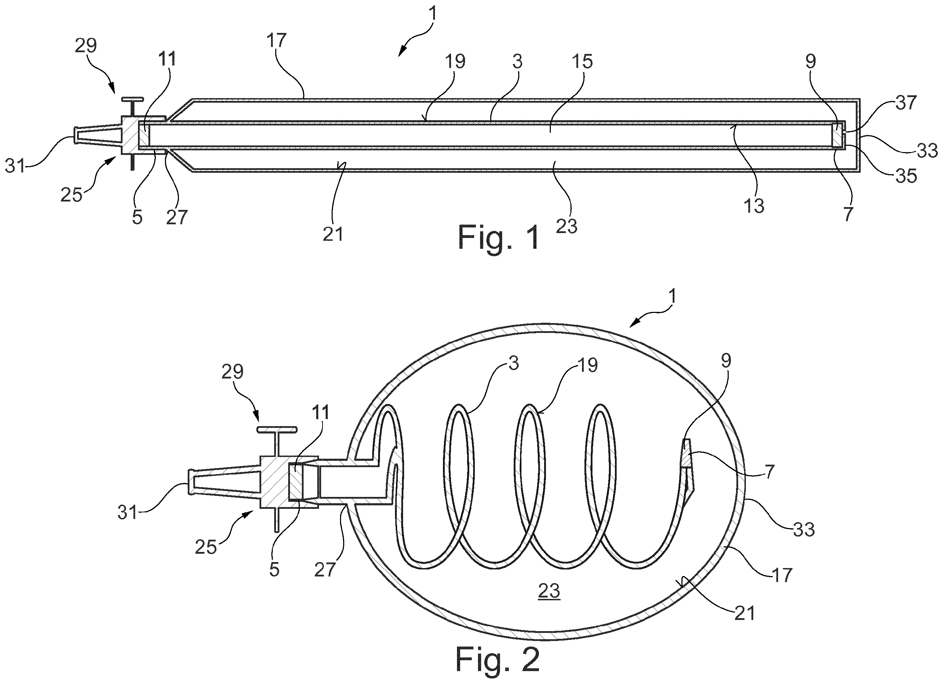

**Fig. 1** zeigt eine schematische Darstellung eines ersten Ausführungsbeispiels eines Behälters 1 für medizinische Flüssigkeiten, der insbesondere als Spritze oder Karpule ausgebildet ist. Der Behälter 1 weist einen Innenbehälter 3 mit einem distalen Ende 5 und einem proximalen Ende 7 auf. An dem proximalen Ende 7 ist ein erstes poröses Trennelement 9 angeordnet, und an dem distalen Ende 5 ist bevorzugt ein zweites poröses Trennelement 11 angeordnet. Das erste poröse Trennelement 9 und das zweite poröse Trennelement 11 begrenzen - gemeinsam mit einer inneren Mantelfläche 13 des Innenbehälters 3 - ein Aufnahmevolumen 15, das eingerichtet ist zur Aufnahme einer medizinischen Flüssigkeit, insbesondere eines flüssigen medizinischen Wirk- und/oder Hilfstoffs.

Der Behälter 1 weist außerdem einen Außenbehälter 17 auf, wobei der Innenbehälter 3 in dem Außenbehälter 17 mit seinem proximalen Ende 9 und zumindest bereichsweise mit dem Aufnahmevolumen 15 angeordnet ist. Der Außenbehälter 17 umgreift dabei den Innenbehälter 3 gasdicht, sodass in einem zwischen einer Außenfläche 19 des Innenbehälters 3, die insbesondere eine äußere Mantelfläche ist, und einer Innenfläche 21 des Außenbehälters 17 angeordneten Periphervolumen 23 ein Gas unter Überdruck - relativ zu einer äußeren Umgebung des Behälters 1 - anordenbar ist.

Das Periphervolumen 23 steht über das erste poröse Trennelement 9 mit dem Aufnahmevolumen 15 in Verbindung, sodass insbesondere der in dem Periphervolumen 23 herrschende Druck an das Aufnahmevolumen 15 übertragen wird.

Ein Auslasskanalabschnitt 25 ist mit dem distalen Ende 5 des Innenbehälters 3 verbunden und zumindest bereichsweise außerhalb des Außenbehälters 17 angeordnet, insbesondere distal zu einer Verbindungsstelle 27, an welcher der Außenbehälter 17 gasdicht mit dem Innenbehälter 3, verbunden, vorzugsweise mit diesem verschmolzen ist.

In dem Auslasskanalabschnitt 25 ist eine Ventileinrichtung 29 angeordnet, die eingerichtet ist, um in einer Offenstellung eine Fluidverbindung zwischen einer distalen Auslassöffnung 31 des Auslasskanalabschnitts 25 und dem Aufnahmevolumen 15 freizugeben, und in einer Schließstellung die Fluidverbindung zwischen der distalen Auslassöffnung 31 und dem Aufnahmevolumen 15 zu sperren.

Bevorzugt sind sowohl der Innenbehälter 3 als auch der Außenbehälter 17 aus Glas gebildet, insbesondere der Innenbehälter 3 aus einem - gegebenenfalls gewundenen -, bei dem Ausführungsbeispiel von Figur 1 geraden, Glasrohr oder Glaszylinder, und der Außenbehälter 17 aus einem Glasrohr, Glaszylinder oder einem Glaskolben, wobei das innere Volumen des Außenbehälters 17 relativ zu dem Innenbehälter 3 so abgestimmt ist, dass der Außenbehälter 17 den Innenbehälter 3 zumindest bereichsweise aufnehmen kann.

Es ist aber auch möglich, dass der Innenbehälter 3 und/oder der Außenbehälter 17 einen Kunststoff aufweisen oder aus Kunststoff gebildet sind. Auch eine Keramik oder ein Metall oder eine Metalllegierung kommen als Material für den Innenbehälter 3 und/oder den Außenbehälter 17 infrage.

Der Innenbehälter 3 ragt vorzugsweise mit seinem proximalen Ende 7 frei in den Außenbehälter 17 hinein und ist insbesondere an dem proximalen Ende 7 nicht mit dem Außenbehälter 17 verbunden. Es ist aber möglich, dass zur mechanischen Unterstützung des Innenbehälters 3 in dem Außenbehälter 17 ein Stützelement, insbesondere ein radialer Steg, oder auch eine Mehrzahl solcher Stützelemente, insbesondere radialer Stege, zwischen dem Innenbehälter 3 und dem Außenbehälter 17 angeordnet sind, welche den Innenbehälter 3 an der Innenfläche 21 des Außenbehälters 17 abstützen. Der Außenbehälter 17 ist an seinem proximalen Ende 33 in einem - in Längsrichtung des Behälters 1 gemessenen - Abstand zu dem proximalen Ende 7 des Innenbehälters 3 geschlossen ausgebildet.

Im Bereich der Verbindungsstelle 27, die in der Nähe des distalen Endes 5 des Innenbehälters 3 angeordnet und vorzugsweise von diesem aus gesehen in proximaler Richtung versetzt angeordnet ist, ist der Außenbehälter 17 mit dem Innenbehälter 3 gasdicht verbunden, insbesondere verschmolzen. Auf diese Weise ist das Periphervolumen 23 allseitig gasdicht durch den Außenbehälter 17 umschlossen.

Es ist auch möglich, dass die Verbindungsstelle 27 - in Längsrichtung des Behälters 1 gesehen - auf Höhe von dessen distalen Ende 5 vorgesehen ist. Grundsätzlich ist es auch möglich, dass das distale Ende 5 des Innenbehälters 3 innerhalb des Außenbehälters 17 angeordnet ist, wobei sich dann der Auslasskanalabschnitt 25 bereichsweise in den Außenbehälter 17 hinein erstreckt, und wobei die Verbindungsstelle 27 dann als unmittelbare Verbindung zwischen dem Außenbehälter 17 und dem Auslasskanalabschnitt 25 ausgebildet ist.

Die Längsrichtung des Behälters 1 ist insbesondere diejenige Richtung, in welcher der Behälter 1 seine längste Erstreckung aufweist, und/oder diejenige Richtung, welche in Richtung einer Symmetrieachse des Behälters 1 weist. In Figur 1 ist dies die horizontale Richtung. Eine radiale Richtung steht senkrecht auf der Längsrichtung. Eine Umfangsrichtung umgreift die Längsrichtung konzentrisch.

Das erste poröse Trennelement 9 schließt den Innenbehälter 3 an seinem proximalen Ende 7 ab. Das zweite poröse Trennelement 11 begrenzt das Aufnahmevolumen 15 im Bereich des distalen Endes 5 des Innenbehälters 3.

Die porösen Trennelemente 9, 11 sind raumfest an dem Innenbehälter 3 und insbesondere raumfest zu der inneren Mantelfläche 13 des Innenbehälters 3 angeordnet, mithin nicht in dem Innenbehälter 3 oder relativ zu dem Innenbehälter 3 verlagerbar. Insbesondere ist es möglich, dass die porösen Trennelemente 9, 11 mit dem Innenbehälter 3 stoffschlüssig verbunden, vorzugsweise verschmolzen sind.

Die in dem Aufnahmevolumen 15 angeordnete medizinische Flüssigkeit kann über die Ventileinrichtung 29 in deren Offenstellung aus der distalen Auslassöffnung 31 abgegeben werden, da das unter Überdruck in dem Periphervolumen 23 angeordnete Gas die medizinische Flüssigkeit vermittelt über das erste poröse Trennelement 9 mit Druck beaufschlagt, der größer ist als der Umgebungsdruck in der äußeren Umgebung des Behälters 1. Die medizinische Flüssigkeit wird daher in der Offenstellung der Ventileinrichtung 29 über die distale Auslassöffnung 31 ausgetrieben. Hierzu bedarf es keinerlei beweglicher Teile, insbesondere keiner verlagerbaren Stopfen, sodass der Behälter 1 stopfenfrei ausgebildet ist, wobei er insbesondere keinen verlagerbaren Stopfen, ganz besonders keinen verlagerbaren Stopfen aus einem pharmazeutischen Gummi, aufweist.

Das Austreiben der medizinischen Flüssigkeit aus dem Aufnahmevolumen 15 kann unterbrochen werden, indem die Ventileinrichtung 29 von ihrer Offenstellung in ihre Schließstellung verlagert wird. Auf diese Weise ist eine Mehrfachapplikation der medizinischen Flüssigkeit möglich, beispielsweise an verschiedenen Stellen bei subkutaner oder intramuskulärer Applikation, oder über einen längeren Zeitraum hinweg.

Das Periphervolumen 23 ist vorzugsweise mindestens so groß wie das Aufnahmevolumen 15, vorzugsweise größer als das Aufnahmevolumen 15. Hierdurch kann eine möglichst homogene Abgabe der medizinischen Flüssigkeit 15 über die distale Auslassöffnung 31 mit möglichst über die gesamte Abgabedauer definierter, gegebenenfalls konstanter oder in bestimmter Weise variierender Flussrate gewährleistet werden.

Der Innenbehälter 3 ist vorzugsweise als Kapillare, insbesondere mit einem Innendurchmesser von weniger als 4 mm, vorzugsweise von weniger als 3 mm, ausgebildet. Hierdurch wird in vorteilhafter Weise die Flüssigkeitssäule der medizinischen Flüssigkeit in dem Aufnahmevolumen 15 zusammengehalten, was zum einen ein Befüllen des Aufnahmevolumens 15 erleichtert und zum anderen bei der Abgabe der medizinischen Flüssigkeit verhindert, dass sich eine Luftblase im Bereich des distalen Endes 5 und somit insbesondere des zweiten porösen Trennelements 11 bilden kann. Eine Luft- oder Gasinjektion kann somit in vorteilhafter Weise vermieden werden. Auch nach vollständiger Abgabe der medizinischen Flüssigkeit aus dem Aufnahmevolumen 15 verhindert das mit der medizinischen Flüssigkeit noch benetzte, zweite poröse Trennelement 11 aufgrund der in diesem wirkenden Kapillarkräfte, dass Gas hindurchdringt und injiziert wird.

Die kapillare Eigenschaft des Innenbehälters 3 ermöglicht auch ein vollständiges Entleeren des Aufnahmevolumens 15, indem nämlich die Kapillarkräfte die Flüssigkeitssäule der medizinischen Flüssigkeit beim Entleeren zusammenhalten, sodass diese nicht an der inneren Mantelfläche 13 haften bleibt, sondern vielmehr vollständig abgegeben wird.

Bei dem in Figur 1 dargestellten, ersten Ausführungsbeispiel des Behälters 1 ist der Innenbehälter 3 langgestreckt, insbesondere gerade und vorzugsweise zylindrisch, insbesondere als Kreiszylinder, ausgebildet.

Der Außenbehälter 17 ist bei dem ersten Ausführungsbeispiel des Behälters 1 gemäß Figur 1 ebenfalls - bis auf den Verbindungsbereich im Bereich der Verbindungsstelle 27 - zylindrisch, vorzugsweise kreiszylindrisch, ausgebildet.

Der Innenbehälter 3 ist insbesondere mit der medizinischen Flüssigkeit gefüllt, wobei der Außenbehälter mit dem Gas unter Überdruck gefüllt ist. Bei dem Gas kann es sich um Luft, aber auch um ein Inertgas, insbesondere Stickstoff, ein Edelgas, oder ein Gemisch aus verschiedenen Gasen, insbesondere Stickstoff und/oder wenigstens einem Edelgas, handeln. Ist in dem Periphervolumen 23 ein sauerstofffreies oder zumindest sauerstoffarmes Gas angeordnet, kann die medizinische Flüssigkeit auch sauerstoffempfindlich sein. Der Behälter 1 ist daher auch zur Langzeitlagerung einer sauerstoffempfindlichen medizinischen Flüssigkeit, beispielsweise von Adrenalin, geeignet. Dies ist bei herkömmlichen medizinischen Behältern mit Stopfen aus pharmazeutischem Gummi typischerweise nicht der Fall, da diese Stopfen eine gewisse Durchlässigkeit für Sauerstoff aufweisen, während der Behälter 1 in der Schließstellung der Ventileinrichtung 29 nach außen gasdicht ist.

Das erste, proximale poröse Trennelement 9 ist hier mit einem Abdeckelement 35 zumindest bereichsweise gegenüber dem Periphervolumen 23 des Außenbehälters 17 abgedeckt, wobei das Abdeckelement das proximale poröse Trennelement 9 bis auf eine zentrale Aussparung 37 abdeckt. Das Abdeckelement 35 kann insbesondere als Kappe mit zentraler Bohrung oder als Membran mit kleiner Öffnung ausgebildet sein. Alternativ ist es auch möglich, dass das erste poröse Trennelement 9 sehr klein, das heißt mit kleinem Durchmesser, ausgebildet ist und in einen radial nach innen aufgedickten Bereich der inneren Mantelfläche 13 angeordnet, insbesondere eingeschmolzen ist. Die derart verringerte Fläche, über welche das erste poröse Trennelement 9 mit dem Gas in dem Periphervolumen 23 in Kontakt steht, vermindert in vorteilhafter Weise einen Übergang zumindest von Teilen der medizinischen Flüssigkeit in die Dampfphase und somit ein Austrocknen des proximalen porösen Trennelements 9.

Im Bereich der Verbindungsstelle 27 ist bevorzugt eine Sollbruchstelle angeordnet. Auf diese Weise kann auch bei einer versehentlichen Schlagbelastung des Behälters 1 dessen explosionsartiges Zerreißen vermieden werden. Vielmehr wird bevorzugt ein kontrolliertes Entweichen des Gases aus dem Periphervolumen 23 - vorzugsweise ohne wesentlichen Austritt der medizinischen Flüssigkeit - ermöglicht. Die Sollbruchstelle ist vorzugsweise als Bohrung oder Loch ausgebildet, die/das quer zur Längsrichtung, insbesondere in radialer Richtung ausgerichtet ist. Dies verhindert, dass der Behälter 1 durch aus dem Periphervolumen 23 entweichendes Gas quasi raketenartig beschleunigt wird. Es ergibt sich so höchstens eine Rotation des Behälters 1 um eine Achse, die senkrecht auf der Längsachse steht, aber keine translatorische Verlagerung. Die Sollbruchstelle erhöht auf diese Weise die Sicherheit des Behälters 1 im Betrieb.

Der Behälter 1 ist vorzugsweise zur Aufnahme kleiner Volumina an medizinischer Flüssigkeit, insbesondere von weniger als 1 ml, vorzugsweise von weniger als 0,6 ml, vorzugsweise von weniger als 0,5 ml, vorzugsweise von 1 ml, eingerichtet. Besonders bevorzugt ist er eingerichtet zur Aufnahme einer medizinischen Flüssigkeit, die für eine ophthalmische Injektion, mithin insbesondere eine Injektion in den Glaskörper eines Auges, vorgesehen ist. Insbesondere in diesem Fall wirkt sich vorteilhaft aus, dass das distale, zweite poröse Trennelement 11 aufgrund seiner porösen Eigenschaften auch eine Filterwirkung entfaltet und somit wirksam eine Partikelinjektion in das Auge verhindern kann.

Das erste poröse Trennelement 9 und/oder das zweite poröse Trennelement 11, besonders bevorzugt beide porösen Trennelemente 9, 11, ist/sind vorzugsweise als Sinterkörper, insbesondere als Fritte, bevorzugt als Glasfritte oder Keramikfritte, oder als Filter oder Filtermembran ausgebildet.

Das distale, zweite poröse Trennelement 11 ist vorzugsweise außerhalb des Außenbehälters 17, hier insbesondere distal zu der Verbindungsstelle 27 versetzt, angeordnet. Es ist aber auch möglich, dass das distale, zweite poröse Trennelement 11 auf Höhe der Verbindungsstelle 27 oder sogar in dem Außenbehälter 17 angeordnet ist, wie zuvor bereits beschrieben.

Der Auslasskanalabschnitt 25 ist hier mehrteilig mit dem Innenbehälter 3 ausgebildet, wobei er insbesondere als Aufsatz, vorzugsweise als Kunststoffaufsatz, ausgebildet ist, der dicht auf das distale Ende 5 des Innenbehälters 3 aufgesetzt und dort insbesondere über geeignete Halte- sowie Dichtmittel dicht gehalten ist. Es ist alternativ aber auch möglich, dass der Auslasskanalabschnitt mit dem Innenbehälter einstückig ausgebildet ist.

Der Auslasskanalabschnitt 25 weist bevorzugt eine geeignete Aussparung, insbesondere eine Querbohrung, zur Aufnahme der Ventileinrichtung 29 auf.

**Fig. 2** zeigt eine schematische Darstellung eines zweiten Ausführungsbeispiels des Behälters 1. Gleiche und funktionsgleiche Elemente sind mit gleichen Bezugszeichen versehen, sodass insofern auf die vorangegangene Beschreibung verwiesen wird. Bei diesem zweiten Ausführungsbeispiel ist der vorzugsweise ebenfalls als Kapillare ausgebildete Innenbehälter 3 gewunden, insbesondere spiralig gewunden ausgebildet. Dies verkürzt bei gleichem Aufnahmevolumen 15 und insbesondere bei gleichem Innendurchmesser des Innenbehälters 3 die Baulänge des Behälters 1.

Der Außenbehälter 17 ist bei dem zweiten Ausführungsbeispiel insbesondere tonnen- oder kolbenförmig, konvex und bevorzugt ovoid oder oval ausgebildet. Insbesondere weist er eine gekrümmte Wandung auf, die in zumindest zwei aufeinander senkrecht stehenden Richtungen eine endliche, von Null verschiedene Krümmung aufweist. Der Außenbehälter 17 ist bei dieser Geometrie besonders druckstabil, insbesondere da er keinerlei Ecken oder scharfe Übergänge aufweist.

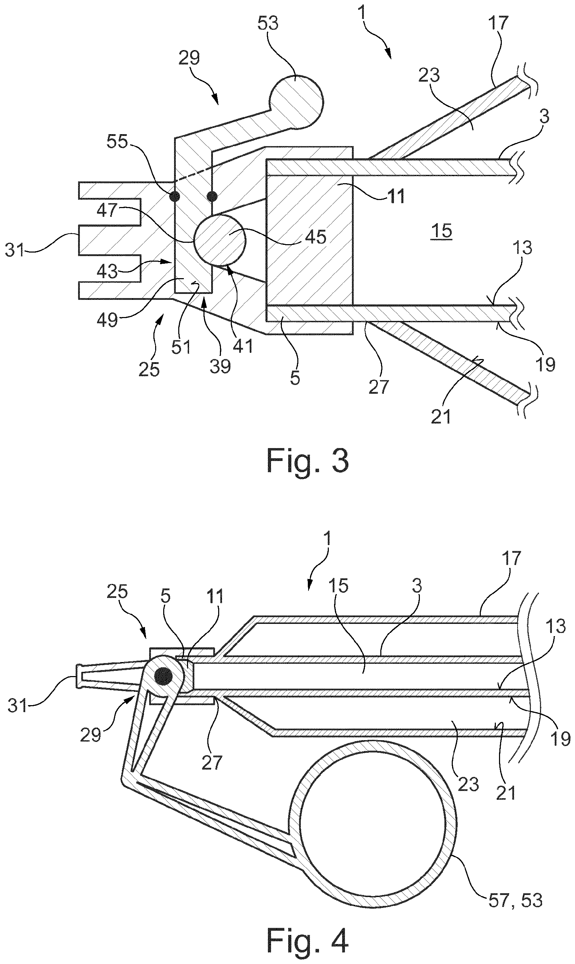

**Fig.** 3 zeigt eine schematische Detaildarstellung eines dritten Ausführungsbeispiels des Behälters 1. Gleiche und funktionsgleiche Elemente sind mit gleichen Bezugszeichen versehen, sodass insofern auf die vorangegangene Beschreibung verwiesen wird. Anhand von Figur 3 wird insbesondere eine bevorzugte Funktionsweise und Ausgestaltung der Ventileinrichtung 29 näher erläutert. Die Ventileinrichtung 29 weist hier ein Kombinationsventil 39 mit einem Rückschlagventil 41 und einem handbetätigten Ventil 43 auf, die integral miteinander als Ventileinrichtung 29 ausgebildet sind. Das Rückschlagventil 41 weist ein Rückschlagventilelement 45 auf, das hier insbesondere kugelförmig, insbesondere als Ventilkugel, ausgebildet ist. Dieses Rückschlagventilelement 45 wird von einem Überdruck in dem Aufnahmevolumen 15 in einen Ventilsitz 47 gedrängt, der zumindest bereichsweise in einem Ventilelement 49 des handbetätigten Ventils 43 ausgebildet ist. Dieses Ventilelement 49 ist vorzugsweise als in dem Auslasskanalabschnitt 25 um eine Ventildrehachse drehbar gelagerter Zylinder ausgebildet, wobei die Ventildrehachse in radiale Richtung weist und somit senkrecht auf der Längsrichtung des Behälters 1 steht. Der Ventilsitz 47 ist in dem Ventilelement 49 als Aussparung oder Mulde ausgebildet, somit als Vertiefung in einer äußeren Umfangsfläche 51 des Ventilelements 49.

Das Ventilelement 49 ist - hier einstückig - mit einem Betätigungselement 53, insbesondere einem Griff, verbunden, wobei das Ventilelement 49 durch das Betätigungselement 53 insbesondere handbetätigt um die Ventildrehachse geschwenkt werden kann.

In Figur 3 ist das Kombinationsventil 39 und damit die Ventileinrichtung 29 in seiner/ihrer Schließstellung dargestellt. Dadurch, dass das Rückschlagventilelement 45 durch den Überdruck in dem Aufnahmevolumen 15 in den Ventilsitz 47 gepresst wird, ist das Aufnahmevolumen 15 gegenüber der distalen Auslassöffnung 31 dicht verschlossen.

Wird das Ventilelement 49 um die Ventildrehachse - insbesondere um 90° - verschwenkt, drängt die äußere Umfangsfläche 51 außerhalb der den Ventilsitz 47 bildenden Aussparung das Rückschlagventilelement 45 in Richtung des Aufnahmevolumens 15, mithin in Figur 3 nach rechts, sodass es aus dem Ventilsitz 47 heraus verlagert wird. Dadurch wird die Sperrwirkung des Rückschlagventilelements 45 aufgehoben. Zugleich stellt nun die den Ventilsitz 47 bildende Aussparung des Ventilelements 49 einen Fluidpfad bereit, über den das Aufnahmevolumen 15 mit der distalen Auslassöffnung 31 in Fluidverbindung ist. Das Kombinationsventil 39 und mithin auch die Ventileinrichtung 29 ist somit in der Offenstellung angeordnet. Eine Verlagerung zurück in die Schließstellung erfolgt in einfacher Weise so, dass das Ventilelement 49 mit dem Ventilsitz 47 wieder in eine Position gebracht wird, in welcher das Rückschlagventilelement 45 dichtend in dem Ventilsitz 47 aufgenommen werden kann, mithin insbesondere in die in Figur 3 dargestellte Position.

Das Ventilelement 49 ist insbesondere über eine Dichtung 55, die hier insbesondere als O-Ring ausgebildet ist, in dem Auslasskanalabschnitt 25 fluiddicht gelagert.

Alternativ zu der hier dargestellten Ausgestaltung kann die Ventileinrichtung 29 auch ein Rückschlagventil, ein handbetätigtes Ventil, oder ein Rückschlagventil und ein handbetätigtes Ventil, die separat voneinander ausgebildet sind, oder ein Schaltventil aufweisen.

**Fig. 4** zeigt eine schematische Darstellung eines vierten Ausführungsbeispiels des Behälters 1. Gleiche und funktionsgleiche Elemente sind mit gleichen Bezugszeichen versehen, sodass insofern auf die vorangegangene Beschreibung verwiesen wird. Bei diesem vierten Ausführungsbeispiel weist der Behälter 1 einen Fingerabzug 57 als Betätigungselement 53 für die Ventileinrichtung 29 auf. Hierdurch ist die Ventileinrichtung 29 auf besonders ergonomische Weise betätigbar, insbesondere wenn ein Patient sich selbst eine Injektion aus dem Behälter 1 verabreicht.

Es wird auch ein nicht dargestelltes Ausführungsbeispiel des Behälters 1 bevorzugt, bei welchem der Auslasskanalabschnitt 25 in einem endlichen, von 0° verschiedenen Winkel zu der Haupterstreckungsrichtung, mithin insbesondere zu der Längsrichtung, des Innenbehälters 3 angeordnet ist. Auf diese Weise kann eine quasi pistolenartige Konfiguration des Behälters 1 bereitgestellt werden, die besonders ergonomisch und insbesondere mit bequemer Handhaltung bedienbar ist. Bevorzugt ist diese pistolenartige Ausgestaltung des Behälters 1 kombiniert mit einem Fingerabzug 57 als Betätigungselement 53 für die Ventileinrichtung 29, was die Ergonomie des Behälters 1 weiter steigert und dessen Bedienbarkeit insbesondere auch für in ihrer Beweglichkeit eingeschränkte Patienten, die sich selbst eine Injektion setzen wollen, erhöht.

Ein Verfahren zum Befüllen des Behälters 1 sieht bevorzugt vor, dass das Periphervolumen 23 mit einem Gas unter einem ersten vorbestimmten Druck, der größer ist als ein erwarteter äußerer Umgebungsdruck bei einer späteren Verwendung des Behälters 1, insbesondere also über einem Normaldruck von insbesondere 1013 mbar liegt, befüllt wird, wobei das Aufnahmevolumen 15 des Innenbehälters 3 danach mit einer medizinischen Flüssigkeit unter einem zweiten vorbestimmten Druck befüllt wird, wobei der zweite vorbestimmte Druck größer ist als der erste vorbestimmte Druck. Dabei bewirkt insbesondere die Kombination aus den hier genannten Druckverhältnissen und den kapillaren Eigenschaften des Aufnahmevolumens 15 eine luftblasenfreie, vollständige Füllung desselben bis zu dem ersten, proximalen porösen Trennelement 9. Anschließend wird bevorzugt die Ventileinrichtung 29 geschlossen, und die Befüllung ist beendet. Im Ergebnis stellt sich der zweite vorbestimmte Druck als Enddruck, mithin als Überdruck in dem Periphervolumen 23 und in dem Aufnahmevolumen 15 ein. Über die Wahl des zweiten vorbestimmten Drucks können somit die Injektionseigenschaften des Behälters 1 und insbesondere zumindest eine initiale Flussrate für einen Austritt der medizinischen Flüssigkeit aus der distalen Auslassöffnung 31 eingestellt werden. Der erste vorbestimmte Druck definiert bevorzugt einen Enddruck zum Ende der Injektion.

Bevorzugt wird vor dem Befüllen des Periphervolumens 23 oder beim Befüllen des Periphervolumens 23 ein Partialdruck zumindest eines Hauptbestandteils der medizinischen Flüssigkeit, insbesondere eines Lösungsmittels, in dem Gas so eingestellt, dass dieser Partialdruck dem Dampfdruck der medizinischen Flüssigkeit oder zumindest des Hauptbestandteils der medizinischen Flüssigkeit in dem Innenbehälter 3 bei dem zweiten vorbestimmten Druck entspricht. Somit wird in dem Periphervolumen 23 ein Sättigungsdampfdruck unter Lagerbedingungen für die medizinische Flüssigkeit eingestellt, sodass ein Verdampfen derselben in das Periphervolumen 23 über das erste poröse Trennelement 9 und damit zugleich ein Austrocknen des ersten porösen Trennelements 9 vermieden wird.

Mit dem hier vorgeschlagenen Behälter 1 wird ein Injektionsbehälter bereitgestellt, der insbesondere für die Selbstanwendung von Patienten besonders geeignet ist. Dabei muss seitens des Bedieners des Behälters 1 kein Pressdruck angewendet werden, um die medizinische Flüssigkeit aus dem Aufnahmevolumen 15 auszutreiben. Dies ermöglicht nicht zuletzt auch die Voreinstellung einer geeigneten Flussrate für das Austreiben der medizinischen Flüssigkeit seitens des Befüllers des Behälters 1.

Der Behälter 1 ist bevorzugt eingerichtet zur Verwendung in einer Autoinjektionseinrichtung, insbesondere einem Autoinjektor oder Pen, oder ist selbst als Autoinjektionseinrichtung ausgebildet.

Dadurch, dass die Ventileinrichtung 29 an dem Auslasskanalabschnitt 25 und somit distal an dem Behälter 1 angeordnet ist, ist sie nahe bei einer zur Injektion vorgesehenen und mit der distalen Auslassöffnung 31 verbundenen Kanüle angeordnet. Es ist für einen Bediener des Behälters 1 auch möglich, diesen nahe an der Ventileinrichtung 29 und dem Auslasskanalabschnitt 25 zu greifen, sodass eine ergonomische Einhand-Bedienung möglich ist.

Der Behälter 1 zeichnet sich insbesondere durch sehr geringe Luft-Totvolumina aus. Ein Luftablassen vor einer Injektion kann daher entfallen.

Da keine verlagerbaren Stopfen vorgesehen sind, bedarf es auch keines Gleitmittels, sodass insbesondere auf eine Verwendung von Silikonöl verzichtet werden kann.

Die Ventileinrichtung 29 ist vorzugsweise in ihre Schließstellung vorgespannt, sodass es einer aktiven Betätigung nur in Richtung der Offenstellung bedarf. Dies vereinfacht die Bedienung des Behälters 1 zusätzlich.

Insbesondere aufgrund der kapillaren Ausgestaltung des Innenbehälters 3 und dessen daher langgestreckter Geometrie ist ein genaues Ablesen auch kleiner Dosierungen ohne weiteres möglich. Dies in Kombination mit der blasenfreien Befüllung und dem Entfall des Erfordernisses, vor einer Injektion Luft aus dem Behälter 1 abzulassen, ermöglicht einen äußerst sparsamen Umgang mit der in dem Aufnahmevolumen 15 angeordneten medizinischen Flüssigkeit. Der medizinische Behälter 1 ist daher nicht nur für kleine Injektionsvolumina, sondern auch zur Verwendung in Zusammenhang mit teuren oder toxischen Substanzen vorteilhaft verwendbar. Der Behälter 1 ermöglicht insbesondere eine ergonomisch günstige und einfache Injektion.

Er eignet sich insbesondere auch für zielgenaue, komplexe Injektionen, insbesondere in der Ophthalmologie und/oder in der Chirurgie.

## Patentansprüche

1. Behälter (1) für die Injektion medizinischer Flüssigkeiten, mit
- einem Innenbehälter (3) mit einem distalen Ende (5) und einem proximalen Ende (7), wobei
- an dem proximalen Ende (7) ein erstes poröses Trennelement (9) angeordnet ist, wobei
- das erste poröse Trennelement (9) ein Aufnahmevolumen (15) des Innenbehälters (3) zur Aufnahme einer medizinischen Flüssigkeit begrenzt, und mit
- einem Außenbehälter (17), in dem der Innenbehälter (3) mit dem proximalen Ende (7) und zumindest bereichsweise mit dem Aufnahmevolumen (15) angeordnet ist, wobei
- der Außenbehälter (17) den Innenbehälter (3) gasdicht umgreift, sodass in einem zwischen einer Außenfläche (19) des Innenbehälters (3) und einer Innenfläche (21) des Außenbehälters (17) angeordneten Periphervolumen (23) ein Gas unter Überdruck anordenbar ist, wobei
- ein Auslasskanalabschnitt (25) mit dem distalen Ende (5) des Innenbehälters (3) verbunden ist, wobei
- der Auslasskanalabschnitt (25) zumindest bereichsweise außerhalb des Außenbehälters (17) angeordnet ist, und wobei
- in dem Auslasskanalabschnitt (25) eine Ventileinrichtung (29) angeordnet ist, die eingerichtet ist, um in einer Offenstellung eine Fluidverbindung zwischen einer distalen Auslassöffnung (31) des Auslasskanalabschnitts (25) und dem Aufnahmevolumen (15) freizugeben, und in einer Schließstellung die Fluidverbindung zwischen der distalen Auslassöffnung (31) und dem Aufnahmevolumen (15) zu sperren.

2. Behälter (1) nach Anspruch 1, **dadurch gekennzeichnet, dass** an dem distalen Ende (5) ein zweites poröses Trennelement (11) angeordnet ist, wobei das erste poröse Trennelement (9) und das zweite poröse Trennelement (11) das Aufnahmevolumen (15) begrenzen.

3. Behälter (1) nach einem der Ansprüche 1 oder 2, **dadurch gekennzeichnet, dass** der Innenbehälter (3)
a) als Kapillare, und/oder
b) langgestreckt, und/oder
c) gerade, insbesondere zylindrisch, insbesondere kreiszylindrisch, und/oder
d) gewunden, insbesondere spiralig,
ausgebildet ist.

4. Behälter (1) nach einem der vorhergehenden Ansprüche, **dadurch gekennzeichnet, dass** der Auslasskanalabschnitt (25) mit dem Innenbehälter (3) einstückig oder mehrteilig, insbesondere als Aufsatz, insbesondere als Kunststoffaufsatz, ausgebildet ist.

5. Behälter (1) nach einem der vorhergehenden Ansprüche, **dadurch gekennzeichnet, dass** das erste poröse Trennelement (9) und/oder das zweite poröse Trennelement (11) als Sinterkörper, insbesondere als Fritte, bevorzugt als Glasfritte oder Keramikfritte, oder als Filter oder Filtermembran ausgebildet ist/sind.

6. Behälter (1) nach einem der vorhergehenden Ansprüche, **dadurch gekennzeichnet, dass** das zweite poröse Trennelement (11) außerhalb des Außenbehälters (17) angeordnet ist.

7. Behälter (1) nach einem der vorhergehenden Ansprüche, **dadurch gekennzeichnet, dass** der Außenbehälter (17)
a) zylindrisch, und/oder
b) tonnen- oder kolbenförmig, und/oder
c) zumindest bereichsweise konvex, vorzugsweise überall konvex, und/oder
d) ovoid oder oval,
ausgebildet ist.

8. Behälter (1) nach einem der vorhergehenden Ansprüche, **dadurch gekennzeichnet, dass** der Auslasskanalabschnitt (25) in einem endlichen, von 0° verschiedenen Winkel zu einer Haupterstreckungsrichtung des Innenbehälters (3) angeordnet ist.

9. Behälter (1) nach einem der vorhergehenden Ansprüche, **dadurch gekennzeichnet, dass** der Behälter (1) einen Fingerabzug (57) aufweist, der mit der Ventileinrichtung (29) derart wirkverbunden ist, dass die Ventileinrichtung (29) mittels des Fingerabzugs (57) betätigbar ist.

10. Behälter (1) nach einem der vorhergehenden Ansprüche, **dadurch gekennzeichnet, dass** die Ventileinrichtung (29)
a) ein Rückschlagventil (41), und/oder
b) ein handbetätigtes Ventil (43), und/oder
c) ein Schaltventil, und/oder
d) ein Kombinationsventil (39) aus einem Rückschlagventil (41) und einem handbetätigten Ventil (43)
aufweist.

11. Behälter (1) nach einem der vorhergehenden Ansprüche, **dadurch gekennzeichnet, dass** der Innenbehälter (3) mit einer medizinischen Flüssigkeit gefüllt ist, wobei der Außenbehälter (17) mit einem Gas unter Überdruck gefüllt ist.

12. Behälter (1) nach einem der vorhergehenden Ansprüche, **dadurch gekennzeichnet, dass** das erste, proximale poröse Trennelement (9) durch ein Abdeckelement (35) zumindest bereichsweise gegenüber dem Periphervolumen (23) des Außenbehälters (17) abgedeckt ist, wobei das Abdeckelement (35) das erste, proximale poröse Trennelement (9) bevorzugt bis auf eine zentrale Aussparung (37) abdeckt.

13. Behälter (1) nach einem der vorhergehenden Ansprüche, **dadurch gekennzeichnet, dass** an einer distalen Verbindungsstelle (27) zwischen dem Außenbehälter (17) und dem Innenbehälter (3) eine Sollbruchstelle angeordnet ist.

14. Behälter (1) nach einem der vorhergehenden Ansprüche, **dadurch gekennzeichnet, dass** der Behälter (1) als Spritze oder Karpule ausgebildet ist.

15. Verfahren zum Befüllen eines Behälters (1) für die Injektion medizinischer Flüssigkeiten nach einem der Ansprüche 1 bis 14, mit folgenden Schritten:
- Befüllen des Periphervolumens (23) des Behälters (1) mit einem Gas unter einem ersten vorbestimmten Druck,
- Befüllen des Aufnahmevolumens (15) des Innenbehälters (13) mit einer medizinischen Flüssigkeit unter einem zweiten vorbestimmten Druck, der größer ist als der erste vorbestimmte Druck, und
- Schließen der Ventileinrichtung (29).

## Claims

1. Container (1) for the injection of medical liquids, comprising
- an inner container (3) with a distal end (5) and a proximal end (7), wherein
- a first porous separating element (9) is arranged at the proximal end (7), wherein
- the first porous separating element (9) delimits a holding volume (15) of the inner container (3) for holding a medical liquid, and comprising
- an outer container (17) in which the inner container (3) is arranged with the proximal end (7) and with at least portions of the holding volume (15), wherein
- the outer container (17) extends around the inner container (3) in a gas-tight manner, such that a gas under positive pressure can be arranged in a peripheral volume (23) between an outer surface (19) of the inner container (3) and an inner surface (21) of the outer container (17), wherein
- an outlet channel section (25) is connected to the distal end (5) of the inner container (3), wherein
- at least portions of the outlet channel section (25) are arranged outside the outer container (17), and wherein
- a valve device (29) is arranged in the outlet channel section (25), which valve device is adapted to open, in an open position, a fluid connection between a distal outlet opening (31) of the outlet channel section (25) and the holding volume (15), and to block, in a closed position, the fluid connection between the distal outlet opening (31) and the holding volume (15).

2. Container (1) according to claim 1, **characterised in that** a second porous separating element (11) is arranged at the distal end (5), wherein the first porous separating element (9) and the second porous separating element (11) delimiting the holding volume (15).

3. Container (1) according any to one of claims 1 or 2, **characterised in that** the inner container (3) is designed
a) as a capillary, and/or
b) elongated, and/or
c) straight, in particular cylindrical, in particular circular cylindrical, and/or
d) drawn, especially spiral.

4. Container (1) according to any one of the preceding claims, **characterised in that** the outlet channel section (25) is formed integrally with the inner container (3), or in several pieces, in particular as an attachment, in particular as a plastic attachment.

5. Container (1) according to any one of the preceding claims, **characterised in that** the first porous separating element (9) and/or the second porous separating element (11) is/are designed as a sintered body, in particular as a frit, preferably as a glass frit or ceramic frit, or as a filter or filter membrane.

6. Container (1) according to any one of the preceding claims, **characterised in that** the second porous separating element (11) is arranged outside the outer container (17).

7. Container (1) according to any one of the preceding claims, **characterised in that** the outer container (17) is designed
a) cylindrical, and/or
b) barrel-shaped or piston-shaped, and/or
c) at least partially convex, preferably completely convex, and/or
d) ovoid or oval.

8. Container (1) according to any one of the preceding claims, **characterised in that** the outlet channel section (25) is arranged at a finite angle other than 0° to a primary direction of extension of the inner container (3).

9. Container (1) according to any one of the preceding claims, **characterised in that** the container (1) has a finger trigger (57) which is operatively connected to the valve device (29) in such a way that the valve device (29) can be actuated by means of the finger trigger (57).

10. Container (1) according to any one of the preceding claims, **characterised in that** the valve device (29) comprises
a) a check valve (41), and/or
b) a manually operated valve (43), and/or
c) a switching valve, and/or
d) a combination valve (39) comprising a check valve (41) and a manually operated valve (43).

11. Container (1) according to any one of the preceding claims, **characterised in that** the inner container (3) is filled with a medical liquid, wherein the outer container (17) being filled with a gas under positive pressure.

12. Container (1) according to any one of the preceding claims, **characterised in that** the first, proximal porous separating element (9) is covered with respect to the peripheral volume (23) of the outer container (17) by a cover element (35) at least in regions, wherein the cover element (35) covers the first, proximal porous separating element (9), preferably except for a central recess (37).

13. Container (1) according to any one of the preceding claims, **characterised in that** a predetermined breaking point is arranged at a distal connection point (27) between the outer container (17) and the inner container (3).

14. Container (1) according to any one of the preceding claims, **characterised in that** the container (1) is designed as a syringe or carpule.

15. Method for filling a container (1) for the injection of medical liquids according to any one of claims 1 to 14, comprising the following steps:
- filling the peripheral volume (23) of the container (1) with a gas at a first predetermined pressure,
- filling the holding volume (15) of the inner container (13) with a medical liquid at a second predetermined pressure which is greater than the first predetermined pressure, and
- closing the valve device (29).

## Revendications

1. Récipient (1) pour l'injection de fluides médicaux, avec
- un récipient interne (3) avec une extrémité distale (5) et une extrémité proximale (7), dans lequel
- un premier élément de séparation poreux (9) est disposé au niveau de l'extrémité proximale (7), dans lequel
- le premier élément de séparation poreux (9) délimite un volume de réception (15) du récipient interne (3) pour la réception d'un fluide médical, et avec
- un récipient externe (17), dans lequel est disposé le récipient interne (3) avec l'extrémité proximale (7) et au moins par endroits avec le volume de réception (15), dans lequel
- le récipient externe (17) entoure le récipient interne (3) de manière étanche aux gaz de telle sorte qu'un gaz en surpression puisse être disposé dans un volume périphérique (23) disposé entre une surface externe (19) du récipient interne (3) et une surface interne (21) du récipient externe (17), dans lequel
- une section de canal de sortie (25) est reliée à l'extrémité distale (5) du récipient interne (3), dans lequel
- la section de canal de sortie (25) est disposée au moins par endroits à l'extérieur du récipient externe (17), et dans lequel
- un dispositif de soupape (29) est disposé dans la section de canal de sortie (25), lequel est conçu, dans une position d'ouverture, pour libérer une communication fluidique entre une ouverture de sortie distale (31) de la section de canal de sortie (25) et le volume de réception (15), et, dans une position de fermeture, pour bloquer la communication fluidique entre l'ouverture de sortie distale (31) et le volume de réception (15).

2. Récipient (1) selon la revendication 1, **caractérisé en ce qu'**un deuxième élément de séparation poreux (11) est disposé au niveau de l'extrémité distale (5), dans lequel le premier élément de séparation poreux (9) et le deuxième élément de séparation poreux (11) délimitent le volume de réception (15).

3. Récipient (1) selon l'une des revendications 1 ou 2, **caractérisé en ce que** le récipient interne (3)
a) se présente sous la forme d'un capillaire, et/ou
b) est de forme allongée, et/ou
c) est droit, en particulier de forme cylindrique, en particulier de forme cylindrique circulaire, et/ou
d) est de forme enroulée, en particulier en spirale.

4. Récipient (1) selon l'une des revendications précédentes, **caractérisé en ce que** la section de canal de sortie (25) est réalisée d'une seule pièce avec le récipient interne (3) ou sous la forme d'une pièce distincte, en particulier sous la forme d'un embout, en particulier sous la forme d'un embout en matière plastique.

5. Récipient (1) selon l'une des revendications précédentes, **caractérisé en ce que** le premier élément de séparation poreux (9) et/ou le deuxième élément de séparation poreux (11) se présentent sous la forme d'un corps fritté, en particulier sous la forme d'une fritte, de préférence sous la forme d'une fritte de verre ou d'une fritte de céramique, ou sous la forme d'un filtre ou d'une membrane de filtre.

6. Récipient (1) selon l'une des revendications précédentes, **caractérisé en ce que** le deuxième élément de séparation poreux (11) est disposé à l'extérieur du récipient externe (17).

7. Récipient (1) selon l'une des revendications précédentes, **caractérisé en ce que** le récipient externe (17) est
a) de forme cylindrique, et/ou
b) en forme de tonneau ou de bulbe, et/ou
c) au moins par endroits convexe, de préférence partout convexe, et/ou
d) de forme ovoïde ou ovale.

8. Récipient (1) selon l'une des revendications précédentes, **caractérisé en ce que** la section de canal de sortie (25) est disposée selon un angle fini différent de 0° par rapport à une direction d'extension principale du récipient interne (3).

9. Récipient (1) selon l'une des revendications précédentes, **caractérisé en ce que** le récipient (1) comprend une gâchette (57), laquelle est en liaison fonctionnelle avec le dispositif de soupape (29) de telle sorte que le dispositif de soupape (29) soit actionnable au moyen de la gâchette (57).

10. Récipient (1) selon l'une des revendications précédentes, **caractérisé en ce que** le dispositif de soupape (29) comprend
a) une soupape antiretour (41), et/ou
b) une soupape à commande manuelle (43), et/ou
c) une soupape de commutation, et/ou
d) une soupape combinée (39) à partir d'une soupape antiretour (41) et d'une soupape à commande manuelle (43).

11. Récipient (1) selon l'une des revendications précédentes, **caractérisé en ce que** le récipient interne (3) est rempli d'un fluide médical, dans lequel le récipient externe (17) est rempli d'un gaz en surpression.

12. Récipient (1) selon l'une des revendications précédentes, **caractérisé en ce que** le premier élément de séparation poreux proximal (9) est recouvert par un élément de recouvrement (35) au moins par endroits par rapport au volume périphérique (23) du récipient externe (17), dans lequel l'élément de recouvrement (35) recouvre le premier élément de séparation poreux proximal (9) de préférence jusqu'à une cavité centrale (37).

13. Récipient (1) selon l'une des revendications précédentes, **caractérisé en ce qu'**un point de rupture est disposé au niveau d'un point de liaison distal (27) entre le récipient externe (17) et le récipient interne (3).

14. Récipient (1) selon l'une des revendications précédentes, **caractérisé en ce que** le récipient (1) se présente sous la forme d'un pulvérisateur ou d'une carpule.

15. Procédé pour le remplissage d'un récipient (1) pour l'injection de fluides médicaux selon l'une des revendications 1 à 14, avec les étapes suivantes :
- remplissage du volume périphérique (23) du récipient (1) avec un gaz sous une première pression prédéterminée,
- remplissage du volume de réception (15) du récipient interne (13) avec un fluide médical sous une deuxième pression prédéterminée, laquelle est supérieure à la première pression prédéterminée, et
- fermeture du dispositif de soupape (29).
